# EUROPEAN PATENT APPLICATION

(11) **EP 4 696 766 A1**
(43) Date of publication of application: **18.02.2026**
(21) Application number: 24788603.9
(22) Date of filing: 29.03.2024
(51) Int. Cl.: C12M 1/21, B01D 19/02, B01D 61/14, C12M 1/02, C12M 1/04

(54) **DEFOAMING DEVICE FOR SUCTIONING AND REMOVING FOAM GENERATED AT UPPER PART OF TREATED LIQUID IN TREATMENT TANK, TREATMENT DEVICE PROVIDED WITH DEFOAMING DEVICE, AND DEFOAMING METHOD USING DEFOAMING DEVICE**

(30) Priority: 10.04.2023 JP 2023063711
(71) Applicant: Mitsubishi Chemical Engineering Corporation, Tokyo 103-0021 (JP)
(72) Inventor: KUNITOMO, Nobuhide, Tokyo 103-0021 (JP); FUJII, So, Tokyo 103-0021 (JP)
(74) Representative: Hasegawa, Kan
(86) International application number: PCT/JP2024/013218
(87) International publication number: WO 2024/214576

(57) **Abstract**

An object of a defoaming device of the present invention, a treatment apparatus including the defoaming device, and a defoaming method using the defoaming device is to provide a defoaming device that does not readily fail and can efficiently remove foam generated at an upper part of a treatment liquid in a treatment tank while ensuring cleanliness and sanitation, a treatment apparatus including the defoaming device, and a defoaming method using the defoaming device. To achieve the object,
one embodiment of a defoaming device according to the present invention has the following features 1) to 4).
1) The defoaming device is disposed outside a lid part or upper mirror part at an upper part of a treatment tank.
2) The defoaming device includes a) a defoaming pipe inside which a high-speed gas stream passes, b) a gas supply pipe through which the high-speed gas stream is supplied to the defoaming pipe, and c) a suction pipe that is connected at one end to the defoaming pipe and at the other end to the lid part or upper mirror part and through which the foam is suctioned from the treatment tank.
3) A gas discharge pipe through which gas is discharged outside and a reflux pipe through which the defoamed treatment liquid is refluxed to the treatment tank are connected to an end of the defoaming pipe on the downstream side of the high-speed gas stream.
4) The high-speed gas stream in the defoaming pipe has a flow rate of 10 m/s or more and 130 m/s or less, and the ratio (a/b) of the inner diameter a of the defoaming pipe to the inner diameter b of the gas supply pipe is 1.1 or more and 4.5 or less.
5) The foam is suctioned from the treatment tank through the suction pipe by means of the ejector action of the high-speed gas stream passing inside the defoaming pipe, and the suctioned foam is removed by means of the shearing action of the high-speed gas stream.

## Description

### Technical Field

The present invention relates to a defoaming device that suctions and removes foam generated at an upper part of a treatment liquid in a treatment tank, a treatment apparatus including the defoaming device, and a defoaming method using the defoaming device. The treatment tank of the present invention is used to process protein-containing dispersion liquids or to perform reactions or treatments such as culture of cells or microorganisms, including aerobic bacteria, anaerobic bacteria, and yeast.

### Background Art

In treatment tanks for processing protein-containing dispersion liquids, such as milk and soy milk, or performing reactions or treatments such as culture of cells or microorganisms, including aerobic bacteria, anaerobic bacteria, and yeast, foam is generated at an upper part of a treatment liquid because of gas generated in the treatment process, a gas blown into the treatment liquid, and agitation with an agitator. For example, in the processing of milk and soy milk, foam is generated in the process tank (treatment tank) because agitation and ventilation causes adsorption of proteins to the interface between air and water to form a film and formation of foam on the surface. In the culture of aerobic bacteria, foam is generated in the culture tank (treatment tank) because air is blown into an aerobic bacteria culture solution. In the fermentation of beer using yeast, which is an anaerobic fungus, carbon dioxide generated by fermentation causes foam in the fermentation tank (treatment tank) to occupy up to 30% of the internal volume of the fermentation tank (process tank) at times.

When a large amount of foam is generated, the treatment tank is filled with foam, and the foam leaks out of the culture tank through the sealing part of the rotation shaft of the agitator, the exhaust pipe, or other parts, resulting in a risk of contamination around the treatment tank, in treatments being conducted simultaneously in adjacent treatment tanks, or in the next batch of culture in the same treatment apparatus. Also, if the amount of the treatment liquid loaded into the treatment tank is reduced to prevent the foam from reaching a lid part or upper mirror part at an upper part of the treatment tank, the treatment efficiency is reduced.

In the related art, defoaming in the treatment tank has involved feeding a defoaming agent, such as a silicone-based agent, into the culture tank or using a mechanical defoaming blade or other tools. The feeding of a defoaming agent, however, causes problems, such as inhibition of treatments and reactions, difficulty in controlling defoaming according to foaming, and contamination of the treatment liquid with impurities. In addition, mechanical defoaming has problems associated with increased equipment complexity and size, and low productivity.

The documents that disclose defoaming in treatment tanks through ultrasonic irradiation include Patent Literature 1 and Patent Literature 2.

In a culture tank of Patent Literature 1 (see FIG. 10), foam 32 generated on the liquid surface in a culture tank 30 for aerobic bacteria is removed in the following manner.
1) The foam 32 generated on the liquid surface of an aerobic bacteria culture solution 31 in the culture tank 30 is discharged through an exhaust pipe 33 connected to an upper part of the culture tank 30.
2) The foam 32 is struck by sonic waves and breaks down when ultrasonic waves are emitted from an ultrasonic horn 35 in response to a foam sensor 34, which is attached to the exhaust pipe 33, sensing foam.
3) The foam that has collapsed and increased in liquid density is separated into gas and liquid by a cyclone 38, and the liquid is refluxed to the culture tank 30 through a reflux liquid pipe 36.

The defoaming method of Patent Literature 1, however, may cause leaking of the foam 32 of the aerobic bacteria culture solution 31 from the sealing part between the rotating shaft of an agitator 37 and the culture tank 30 and from the exhaust pipe 33 connected to the upper part of the culture tank 30. The leaked foam 32 may contaminate the area around the culture tank 30 and the sealing part, which is difficult to sterilize, and may result in contamination in cultures being conducted simultaneously in adjacent culture devices or in the next batch of culture in the same culture device.

In a microorganism culture device of Patent Literature 2 (see FIG. 11), a lid part or upper mirror part 41a of a culture tank 41 has an ultrasonic irradiation unit 45 that emits ultrasonic waves to foam 44 generated on the liquid surface of a microorganism culture solution 42. The ultrasonic irradiation unit 45, however, has a narrow defoaming range and does not exhibit high defoaming performance, and thus the ultrasonic irradiation unit 45 fails to adequately break a large amount of highly viscous foam.

Also, Patent Literatures 3 and 4 disclose that foam generated in a steel strip washing tank is suctioned and discharged by using the power of high-pressure fluid.

In a defoaming device of Patent Literature 3 (see FIG. 12), foam 518 generated in a washing tank 55 for a steel strip 51 is suctioned and discharged from the washing tank 55 by a steam ejector 59, and the discharged foam 518 is sprayed toward a defoaming plate 511 in a defoaming box 510 so that the foam 518 collid partes with the defoaming plate 511 and becomes liquid, whereby the foam 518 is removed. The washing of the steel strip 51 in Patent Literature 3, however, a) does not require cleanliness and sanitation expected in the treatment with a treatment liquid in the treatment tank, b) generates the foam 518, which is fragile foam generated by an alkaline cleaning solution, and c) uses a defoaming method involving removing foam by spraying the discharged foam 518 toward the defoaming plate 511 in the defoaming box 510 so that the foam 518 collid partes with the defoaming plate 511 and becomes liquid, resulting in a large facility and cleanliness and sanitation problems.

A defoaming device of Patent Literature 4 (see FIG. 13) is a defoaming device 6101 that is installed in a washing tank 613 and removes foam 617 generated in the washing tank 613 for a steel strip 612. In the defoaming device 6101, the foam 617 is suctioned from a suction pipe 63, sheared, broken down, and liquefied by using the high-speed gas stream blown into a defoaming pipe 64 from a gas supply pipe 61 and separated into gas and liquid in a gas-liquid separator. The washing of the steel strip 612 in Patent Literature 4, however, a) does not require cleanliness and sanitation expected for the treatment liquid in the treatment tank of the present invention, b) generates the foam 617, which is fragile foam generated by a saponification product of rolling oil and an alkaline liquid (e.g., caustic soda or sodium orthosilicate), and also c) uses the defoaming device 6101 installed in the washing tank 613, resulting in cleanliness and sanitation problems.

### Background Art Literature

### Patent Literature

Patent Literature 1: Japanese Unexamined Patent Application Publication No. 2002-51763
Patent Literature 2: Japanese Unexamined Patent Application Publication No. 2020-130078
Patent Literature 3: Japanese Unexamined Patent Application Publication No. 11-36090
Patent Literature 4: Japanese Unexamined Patent Application Publication No. 2007-319790

### Summary of the Invention

### Problems to Be Solved by the Invention

An object of a defoaming device that suctions and removes foam generated at the upper part of a treatment liquid in a treatment tank, a treatment apparatus including the defoaming device, and a defoaming method using the defoaming device according to the present invention (hereinafter referred to as "defoaming device of the present invention," "treatment apparatus of the present invention," and "defoaming method of the present invention," respectively) is to provide a defoaming device that does not readily fail and can efficiently remove even firm foam (foam with high viscosity, elasticity, and low fluidity) containing proteins, carbohydrates, lipids, and other components and generated at an upper part of a treatment liquid in a treatment tank, while ensuring cleanliness and sanitation, a treatment apparatus including the defoaming device, and a defoaming method using the defoaming device.

### Means for Solving the Problems

[1] A defoaming device that suctions and removes foam generated at an upper part of a treatment liquid in a treatment tank, characterized in that
   the defoaming device is disposed outside a lid part or upper mirror part at an upper part of the treatment tank,
   the defoaming device includes a) a defoaming pipe inside which a high-speed gas stream passes, b) a gas supply pipe through which the high-speed gas stream is supplied to the defoaming pipe, and c) a suction pipe that is connected at one end to the defoaming pipe and at the other end to the lid part or upper mirror part and through which the foam is suctioned from the treatment tank,
   the high-speed gas stream in the defoaming pipe has a flow rate of 10 m/s or more and 130 m/s or less, and a ratio (a/b) of an inner diameter a of the defoaming pipe to an inner diameter b of the gas supply pipe is 1.1 or more and 4.5 or less,
   a gas discharge pipe through which gas is discharged outside and a reflux pipe through which the defoamed treatment liquid is refluxed to the treatment tank are connected to an end of the defoaming pipe on a downstream side of the high-speed gas stream, and
   the foam is suctioned from the treatment tank through the suction pipe by means of an ejector action of the high-speed gas stream passing inside the defoaming pipe, and the suctioned foam is broken down by means of a shearing action of the high-speed gas stream.
[2] The defoaming device according to [1], characterized in that an end of the gas supply pipe on the downstream side of the high-speed gas stream is disposed so as to protrude on a further downstream side of the high-speed gas stream than is a connection opening of the suction pipe to the defoaming pipe.
[3] A defoaming device that suctions and removes foam generated at an upper part of a treatment liquid in a treatment tank, characterized in that
   the defoaming device is disposed outside a lid part or upper mirror part at an upper part of the treatment tank,
   the defoaming device includes a) a defoaming pipe inside which a high-speed gas stream passes, b) a gas supply pipe through which the high-speed gas stream is supplied to the defoaming pipe, and c) a suction pipe that is connected at one end to the defoaming pipe and at the other end to the lid part or upper mirror part and through which the foam is suctioned from the treatment tank,
   a gas discharge pipe through which gas is discharged outside and a reflux pipe through which the defoamed treatment liquid is refluxed to the treatment tank are connected to an end of the defoaming pipe on a downstream side of the high-speed gas stream,
   a central portion of a connection part of the defoaming pipe to the suction pipe has a cylindrical shape in a flow direction of the high-speed gas stream, and both outer side portions of the connection part have conical shapes that gradually expand in diameter toward upstream and downstream sides of the high-speed gas stream,
   the high-speed gas stream in the defoaming pipe has a flow rate of 10 m/s or more and 130 m/s or less, and a ratio (a/c) of an inner diameter a of the defoaming pipe to an inner diameter c of the connection part is 1.1 or more, and
   the foam is suctioned from the treatment tank inside the suction pipe by means of an ejector action of the high-speed gas stream passing through the defoaming pipe, and the suctioned foam is broken down by means of a shearing action of the high-speed gas stream.
[4] The defoaming device according to any one of [1] to [3], characterized in that an extension pipe that extends the suction pipe toward a liquid surface of the treatment liquid is connected to the suction pipe.
[5] The defoaming device according to any one of [1] to [4], characterized in that the gas discharge pipe has a mist separator that prevents droplets from being released outside.
[6] The defoaming device according to any one of [1] to [5], characterized in that a foreign substance discharge pipe through which foreign substances are discharged outside is connected to the reflux pipe.
[7] The defoaming device according to any one of [1] to [6], characterized in that a separator that separates foreign substances from the defoamed treatment liquid is disposed in the reflux pipe, and a second reflux pipe through which the defoamed treatment liquid from which the foreign substances have been separated and removed is returned to the treatment tank and a foreign substance discharge pipe through which the foreign substances are discharged outside are connected to the separator.
[8] The defoaming device according to any one of [1] to [7], characterized in that the treatment liquid is a culture solution in which microorganisms or cells are cultured.
[9] The defoaming device according to any one of [1] to [7], characterized in that the treatment liquid is a culture solution in which aerobic bacteria are cultured, and the high-speed gas stream contains oxygen.
[10] The defoaming device according to any one of [1] to [7], characterized in that the treatment liquid is a culture solution in which anaerobic bacteria are cultured, and the high-speed gas stream has a low oxygen content.
[11] The defoaming device according to any one of [1] to [7], characterized in that the treatment liquid is a culture solution in which yeast is cultured to produce beer, and the high-speed gas stream contains carbon dioxide.
[12] The defoaming device according to any one of [1] to [7], characterized in that the treatment liquid is a protein-containing dispersion liquid, and the high-speed gas stream has a low oxygen content.
[13] A treatment apparatus comprising a treatment tank that accommodates and treats a treatment liquid, characterized in that the defoaming device according to any one of [1] to [12] is disposed outside a lid part or upper mirror part of the treatment tank.
[14] The treatment apparatus according to [13], characterized in that the treatment tank has an agitator for agitating the treatment liquid.
[15] The treatment apparatus according to [13] or [14], characterized in that the treatment apparatus includes a) an extraction conduit through which the treatment liquid is extracted from the treatment tank and supplied to a filter, b) a recovery conduit through which a filtrate separated by the filter is recovered, and c) a reflux conduit through which a concentrate separated by the filter and free of the filtrate is refluxed to the treatment tank.
[16] A treatment apparatus comprising a primary treatment tank that accommodates and treats a treatment liquid and a secondary treatment tank that treats a filtrate obtained by extracting the treatment liquid from the primary treatment tank and filtering the extracted treatment liquid, characterized in that the defoaming device according to any one of [1] to [12] is disposed outside a lid part or upper mirror part of the secondary treatment tank.
[17] The treatment apparatus according to [16], characterized in that the primary treatment tank has an agitator for agitating the treatment liquid.
[18] A defoaming method for removing foam generated at an upper part of a treatment liquid in a treatment tank, characterized by comprising using the defoaming device according to any one of [1] to [12] disposed outside a lid part or upper mirror part of the treatment tank to remove the foam generated at the upper part of the treatment liquid.

To achieve the above object, a defoaming device that suctions and removes foam generated at an upper part of a treatment liquid in a treatment tank according to the present invention has the following features 1) to 4).
1) The defoaming device is disposed outside a lid part or upper mirror part at an upper part of the treatment tank.
2) The defoaming device includes a) a defoaming pipe inside which a high-speed gas stream passes, b) a gas supply pipe through which the high-speed gas stream is supplied to the defoaming pipe, and c) a suction pipe that is connected at one end to the defoaming pipe and at the other end to the lid part or upper mirror part and through which the foam is suctioned from the treatment tank.
3) A gas discharge pipe through which gas is discharged outside and a reflux pipe through which the defoamed treatment liquid is refluxed to the treatment tank are connected to an end of the defoaming pipe on the downstream side of the high-speed gas stream.
4) The foam is suctioned from the treatment tank inside the suction pipe by means of the ejector action of the high-speed gas stream passing through the defoaming pipe, and the suctioned foam is broken down by means of the shearing action of the high-speed gas stream.

### Effects of the Invention

The defoaming device, treatment apparatus, and defoaming method of the present invention exhibit the following excellent effects 5) to 7) owing to the above defoaming device.
5) Cleanliness and sanitation required in the treatment of the treatment liquid in the treatment tank can be ensured by disposing the defoaming device outside the treatment tank as described above in 1).
6) The foam generated at an upper part of the treatment liquid in the treatment tank can be suctioned not locally but entirely by disposing the defoaming device outside the treatment tank and suctioning the foam from the treatment tank through the suction pipe connected to the lid part or upper mirror part at the upper part of the treatment tank as described above in 1) and 2).
7) A defoaming device that has no mechanical movable parts and does not readily fail can be provided when the foam is suctioned from the treatment tank through the suction pipe by means of the ejector action of the high-speed gas stream passing through the defoaming pipe, and the suctioned foam is broken down by means of the shearing action of the high-speed gas stream, as described above in 2) and 4).

Further, when the high-speed gas stream in the defoaming pipe has a flow rate of 10 m/s or more and 130 m/s or less, and the ratio (a/b) of the inner diameter a of the defoaming pipe to the inner diameter b of the gas supply pipe is 1.1 or more and 4.5 or less as in the embodiment of Claim 1 (first embodiment), or when the high-speed gas stream in the defoaming pipe has a flow rate of 10 m/s or more and 130 m/s or less, and the ratio (a/c) of the inner diameter a of the defoaming pipe to the inner diameter c of the connection part is 1.1 or more as in the embodiment of Claim 3 (second embodiment), the foam generated at the upper part of the treatment liquid in the treatment tank can be suctioned from the treatment tank through the suction pipe by means of the ejector action of the high-speed gas stream passing through the defoaming pipe, and the suctioned foam can be immediately broken down by means of the shearing action of the high-speed gas stream, even if the foam generated at the upper part of the treatment liquid in the treatment tank is firm foam (foam with high viscosity, elasticity, and low fluidity) containing proteins, carbohydrates, lipids, and other components. Therefore, without using the "defoaming plate 511" as in Patent Literature 3 or a "gas-liquid separator" as in Patent Literature 4, the defoamed treatment liquid can be separated from the high-speed gas stream, the gas can be discharged outside through the gas discharge pipe, and the defoamed treatment liquid can be refluxed to the treatment tank through the reflux pipe. In addition, by setting the high-speed gas stream in the defoaming pipe to a specific range and setting the ratio of the inner diameter of the defoaming pipe to the inner diameter of the gas supply pipe to a specific range, the foam can be adequately removed by the defoaming device even with a simplified structure without having auxiliary equipment such as the defoaming box in Patent Literature 3 and the gas-liquid separator in Patent Literature 4.

### Brief Description of the Drawings

[Fig. 1] is a schematic cross-sectional view of a defoaming device according to a first embodiment of the present invention attached to a culture tank.
[Fig. 2] is an enlarged schematic cross-sectional view of the defoaming device of Fig. 1.
[FIG. 3] is a schematic cross-sectional view of a defoaming device according to a second embodiment of the present invention attached to a culture tank.
[FIG. 4] is an enlarged schematic cross-sectional view of the defoaming device of FIG. 3.
[FIG. 5] is a schematic view of the appearance of a reflux pipe connected to a foreign substance discharge pipe according to an embodiment of the defoaming device of the present invention.
[FIG. 6] is a schematic view of the appearance of a reflux pipe connected to a separator, a second reflux pipe, and a foreign substance discharge pipe according to an embodiment of the defoaming device of the present invention.
[FIG. 7] is a schematic cross-sectional view of a beer fermentation tank to which the defoaming device of the present invention can be suitably attached.
[FIG. 8] is a schematic cross-sectional view of a first embodiment of a treatment apparatus of the present invention.
[FIG. 9] is a schematic cross-sectional view of a second embodiment of a treatment apparatus of the present invention.

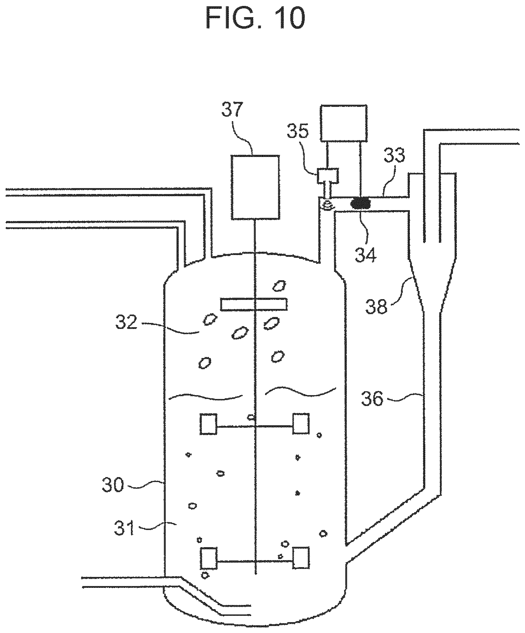

[FIG. 10] is FIG. 2 of Patent Literature 1.

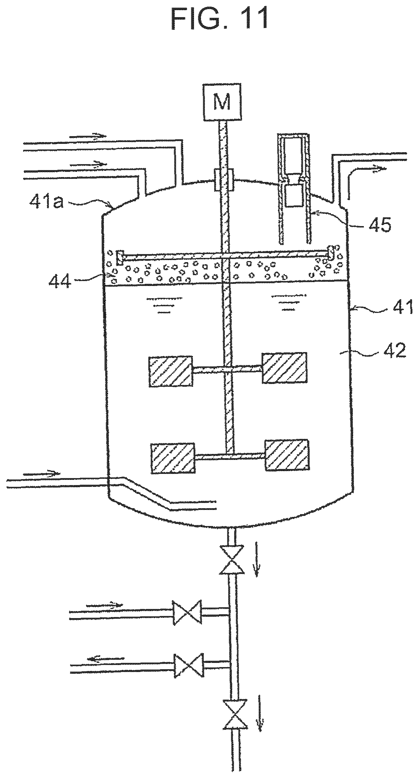

[FIG. 11] is FIG. 1 of Patent Literature 2.

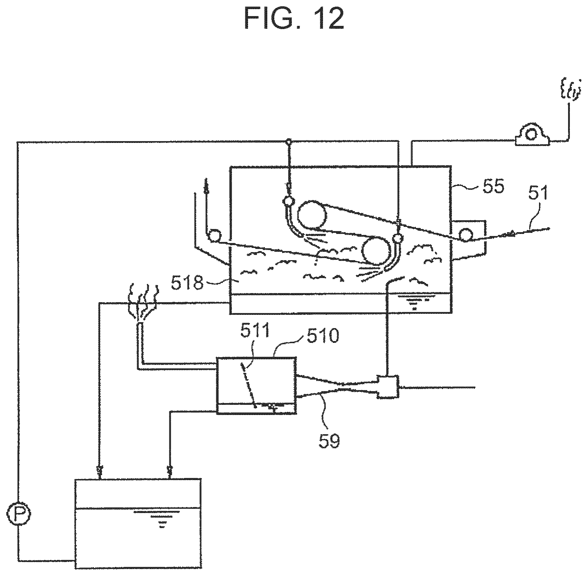

[FIG. 12] is FIG. 1 of Patent Literature 3.

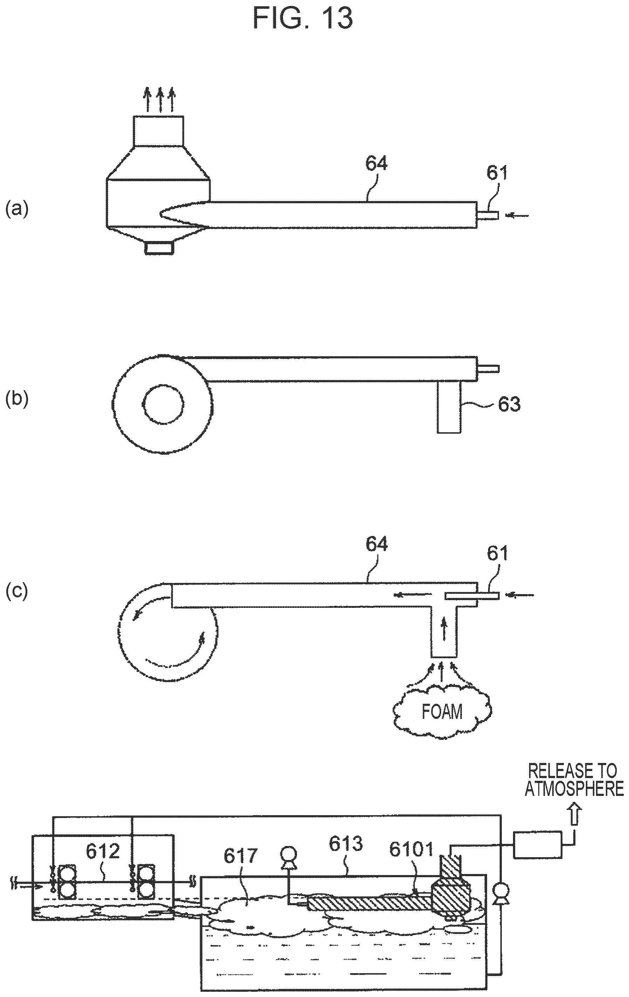

[FIG. 13] includes FIG. 1 and FIG. 2 of Patent Literature 4.

### Mode for Carrying Out the Invention

Embodiments of the present invention will be described below in detail with reference also to the accompanying drawings, but the present invention is not limited to the Embodiments. Also, in the following description, culture tanks (treatment tanks) for culturing microorganisms, such as aerobic bacteria, anaerobic bacteria, and yeast, and process tanks (treatment tanks) for processing milk, soy milk, and other products will be mainly described as treatment tanks to which the defoaming device is attached. The defoaming device of the present invention can also be attached to general treatment tanks for reactions and treatments involving generation of foam.

### <General Defoaming Device of Present Invention>

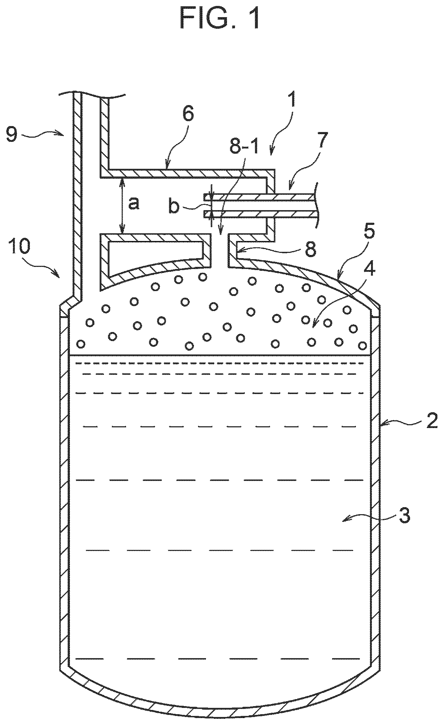

First, a general defoaming device of the present invention will be described with reference to FIG. 1 to FIG. 4.

As illustrated in FIG. 1 to FIG. 4, the defoaming device 1 of the present invention is disposed outside a lid part or upper mirror part 5 at an upper part of a treatment tank 2. Cleanliness and sanitation are required in the culture of microorganisms, such as aerobic bacteria, anaerobic bacteria, and yeast, and the processing of milk, soy milk, and other products. The cleanliness and sanitation of a treatment liquid 3 can be ensured by disposing the defoaming device 1 outside the treatment tank 2. Furthermore, foam 4 generated at an upper part of the treatment liquid 3 in the treatment tank 2 can be efficiently suctioned not locally but entirely by suctioning the foam 4 from the treatment tank 2 through a suction pipe 8 connected to the lid part or upper mirror part 5 at the upper part of the treatment tank 2.

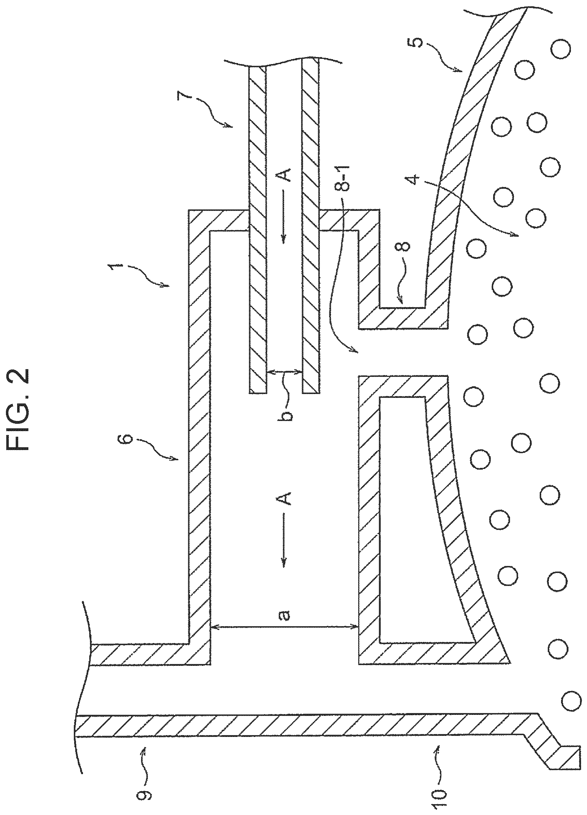

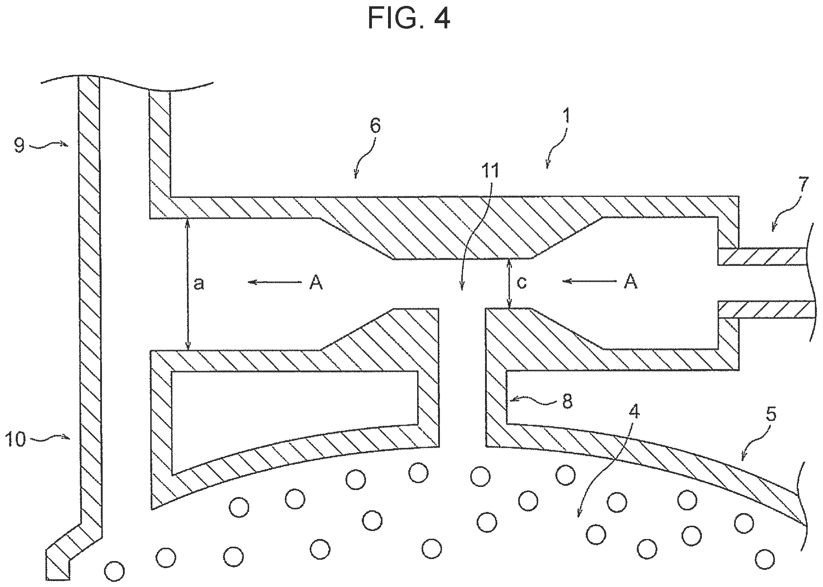

Also, as illustrated enlarged in FIG. 2 and FIG. 4, in the defoaming device 1 of the present invention, a high-speed gas stream A is supplied to the defoaming pipe 6 from the gas supply pipe 7 so that the high-speed gas stream A passes inside the defoaming pipe 6. The foam 4 is suctioned from the treatment tank 2 by means of the ejector action, and the suctioned foam 4 is sheared by means of the shearing action to break down the foam 4, whereby a defoaming device that has no mechanical movable parts and does not readily fail can be provided. To smoothly perform the suction and removal of the foam 4 and other operations, the defoaming device 1 is preferably disposed in a horizontal direction outside the lid part or upper mirror part 5 at the upper part of the treatment tank 2.

Also, in the defoaming device 1 of the present invention, as illustrated in FIG. 1 to FIG. 4, a gas discharge pipe 9 through which the high-speed gas stream A is discharged outside and a reflux pipe 10 through which the defoamed treatment liquid 3 is refluxed to the treatment tank 2 are connected to an end of the defoaming pipe 6 on the downstream side of the high-speed gas stream A.

It should be noted that the cross-sectional shape, diameter expansion and diameter reduction, and other features of the suction pipe 8, the defoaming pipe 6, the gas supply pipe 7, and other pipes can be appropriately designed and set so as to suitably and appropriately perform the suction of the foam 4 from the treatment tank 2, the removal of the foam 4 suctioned by means of the shearing action, and other operations.

### <First Embodiment of Defoaming Device of Present Invention>

In a first embodiment of the defoaming device of the present invention, as illustrated in FIG. 1 and FIG. 2, an end of the gas supply pipe 7 on the downstream side of the high-speed gas stream A is disposed in the defoaming pipe 6 so as to protrude on the further downstream side of the high-speed gas stream A than does a connection opening 8-1 of the suction pipe 8 to the defoaming pipe 6. This configuration prevents the high-speed gas stream A from being blown into the suction pipe 8.

Also, the flow rate of the high-speed gas stream A in the defoaming pipe 6 (i.e., the flow rate of the high-speed gas stream A blown in from the gas supply pipe 7 and passing through the defoaming pipe 6 with an inner diameter a) is 10 m/s or more and 130 m/s or less, and the ratio (a/b) of the inner diameter a of the defoaming pipe 6 to the inner diameter b of the gas supply pipe 7 is 1.1 or more and 4.5 or less.

The lower limit of the flow rate of the high-speed gas stream A in the defoaming pipe 6 is set to 10 m/s or more, preferably 15 m/s or more, more preferably 30 m/s or more from the viewpoint of immediately removing even firm foam (foam with high viscosity, elasticity, and low fluidity) containing proteins, carbohydrates, lipids, and other components. The upper limit of the flow rate of the high-speed gas stream A in the defoaming pipe 6 is set to 130 m/s or less, preferably 90 m/s or less, more preferably 70 m/s or less from the viewpoint of preventing the defoamed treatment liquid from being entrained as droplets in the high-speed gas stream A.

The lower limit of the ratio (a/b) of the inner diameter a of the defoaming pipe 6 to the inner diameter b of the gas supply pipe 7 is 1.1 or more, preferably 1.5 or more, more preferably 1.7 or more, still more preferably 1.8 or more, yet still more preferably 2.0 or more from the viewpoint of sufficiently suctioning even firm foam (foam with high viscosity, elasticity, and low fluidity) containing proteins, carbohydrates, lipids, and other components.

Also, the upper limit of the ratio (a/b) of the inner diameter a of the defoaming pipe 6 to the inner diameter b of the gas supply pipe 7 is 4.5 or less, preferably 4.0 or less, more preferably 3.5 or less, still more preferably 3.0 or less from the viewpoint of sufficiently suctioning even firm foam (foam with high viscosity, elasticity, and low fluidity) containing proteins, carbohydrates, lipids, and other components. When the upper limit of a/b is set to the above upper limit or less, the high-speed gas stream A can be sufficiently expanded in diameter in the defoaming pipe 6, an adequate negative pressure can be generated near the suction pipe 8, and firm foam (foam with high viscosity, elasticity, and low fluidity) containing proteins, carbohydrates, lipids, and other components can be sufficiently suctioned.

Also, the end of the gas supply pipe 7 on the downstream side of the high-speed gas stream is located on the further downstream side of the high-speed gas stream than is the end of the connection opening of the suction pipe 8 to the defoaming pipe 6 on the upstream side of the high-speed gas stream. This configuration allows the foam 4 to be sufficiently suctioned from the treatment tank 2. In FIG. 1 and FIG. 2, the end of the gas supply pipe 7 on the downstream side of the high-speed gas stream is disposed so as to protrude on the further downstream side of the high-speed gas stream than the connection opening of the suction pipe 8 to the defoaming pipe 6. When the end of the gas supply pipe 7 on the downstream side of the high-speed gas stream is located on the further downstream side of the high-speed gas stream than is the end of the connection opening of the suction pipe 8 to the defoaming pipe 6 on the upstream side of the high-speed gas stream, the foam 4 can be sufficiently suctioned from the treatment tank 2 by means of the ejector action of the high-speed gas stream passing inside the defoaming pipe 6.

### <Second Embodiment of Defoaming Device of Present Invention>

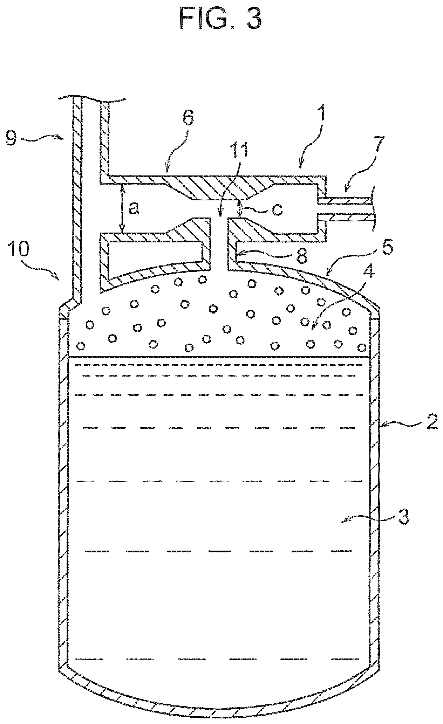

In a second embodiment of the defoaming device of the present invention, as illustrated in FIG. 3 to FIG. 4, a central portion of a connection part 11 of a defoaming pipe 6 to a suction pipe 8 has a cylindrical shape in the flow direction of the high-speed gas stream A, and both ends of the connection part 11 have conical shapes that gradually expand in diameter toward the upstream and downstream sides of the high-speed gas stream. The flow rate of the high-speed gas stream A in the defoaming pipe 6 (i.e., the flow rate of the high-speed gas stream A passing through the defoaming pipe 6 with expanded inner diameter a after passing through the connection part 11) is 10 m/s or more and 130 m/s or less, and the ratio (a/c) of the inner diameter a of the defoaming pipe 6 to the inner diameter c of the connection part 11 is 1.1 or more.

The lower limit of the flow rate of the high-speed gas stream A in the defoaming pipe 6 is set to 10 m/s or more, preferably 15 m/s or more, more preferably 30 m/s or more from the viewpoint of immediately removing even firm foam (foam with high viscosity, elasticity, and low fluidity) containing proteins, carbohydrates, lipids, and other components. The upper limit of the flow rate of the high-speed gas stream A in the defoaming pipe 6 is set to 130 m/s or less, preferably 90 m/s or less, more preferably 70 m/s or less from the viewpoint of preventing the defoamed treatment liquid from being entrained as droplets in the high-speed gas stream A.

The lower limit of the ratio (a/c) of the inner diameter a of the defoaming pipe 6 to the inner diameter c of the connection part 11 is 1.1 or more, preferably 1.5 or more, more preferably 1.7 or more, still more preferably 1.8 or more, yet still more preferably 2.0 or more from the viewpoint of sufficiently suctioning even firm foam (foam with high viscosity, elasticity, and low fluidity) containing proteins, carbohydrates, lipids, and other components.

The upper limit of the ratio (a/c) of the inner diameter a of the defoaming pipe 6 to the inner diameter c of the connection part 11 is preferably 4.5 or less, more preferably 4.0 or less, still more preferably 3.5 or less, yet still more preferably 3.0 or less from the viewpoint of sufficiently suctioning even firm foam (foam with high viscosity, elasticity, and low fluidity) containing proteins, carbohydrates, lipids, and other components.

### <Modification 1 of Defoaming Device of Present Invention>

In the first and second embodiments of the defoaming device of the present invention as illustrated in FIG. 1 to FIG. 4, the defoaming device 1 is disposed at one location outside the treatment tank 2, and the foam 4 that has reached the lid part or upper mirror part 5 is suctioned through the suction pipe 8 connected to a central portion of the lid part or upper mirror part 5 at the upper part of the treatment tank 2, whereby the foam 4 generated at the upper part of the treatment liquid 3 in the treatment tank 2 can be suctioned not locally but entirely.

On the other hand, in a case where the foam 4 is required to be suctioned before the foam 4 reaches the lid part or upper mirror part 5, an extension pipe (not shown) that extends the suction pipe 8 toward the liquid surface of the treatment liquid 3 is connected to the suction pipe 8 to manage such a case.

Also, in a case where a large amount of the foam 4 is generated temporarily or constantly in the process of treating the treatment liquid 3, and a single defoaming device 1 disposed at one location outside the treatment tank 2 is not sufficient to remove the foam, multiple defoaming devices 1 can be disposed at multiple locations at the lid part or upper mirror part 5 to handle such a case. A necessary number of the defoaming devices 1 can be operated at required locations depending on the degree and situation of generation of the foam 4.

### <Modification 2 of Defoaming Device of Present Invention>

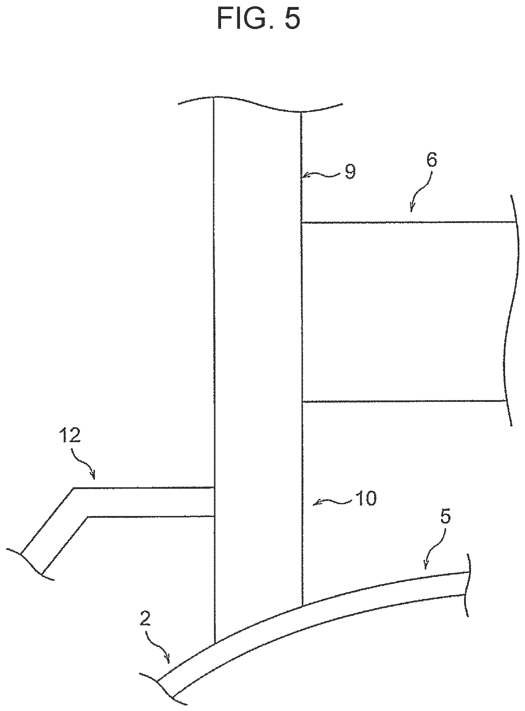

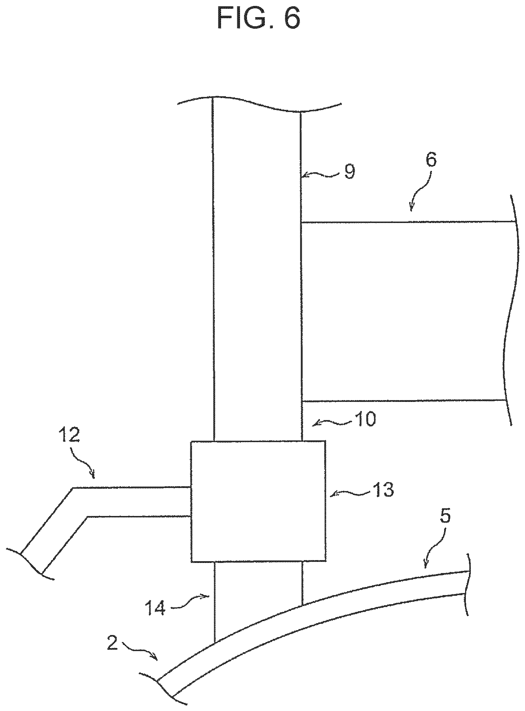

In the defoaming device of the present invention, as illustrated in FIG. 5 and FIG. 6, a gas discharge pipe 9 through which the high-speed gas stream A is discharged outside and a reflux pipe 10 through which the defoamed treatment liquid 3 is refluxed to the treatment tank 2 are connected to an end of the defoaming pipe 6 on the downstream side of the high-speed gas stream A. To prevent droplets of the defoamed treatment liquid 3 entrained in the high-speed gas stream A from being released outside, the gas discharge pipe 9 preferably has a mist separator (not shown). The removal of droplets of the defoamed treatment liquid 3 can, for example, protect the surrounding environment, can prevent contamination, and allows reuse of the gas. A wire mesh or other demisters, and a cyclone or other centrifugal separators can be used as a mist separator.

### <Modification 3 of Defoaming Device of Present Invention>

As illustrated in FIG. 5 and FIG. 6, a foreign substance discharge pipe 12 through which the foreign substances contained in the defoamed treatment liquid 3 are discharged outside is preferably connected to a reflux pipe 10 of the defoaming device of the present invention. The foreign substances, such as dregs, contained in the treatment liquid 3 adhere to the surface of the foam 4 and are suctioned by the defoaming device 1 and incorporated into the defoamed treatment liquid 3 (see FIG. 1 to FIG. 4).

In general, since a large amount of foreign substances is typically contained in the defoamed treatment liquid 3 in the initial stage of treatment, as illustrated in FIG. 5, the defoamed treatment liquid 3 is discharged outside through the foreign substance discharge pipe 12 during the initial stage of treatment, and the defoamed treatment liquid 3 is refluxed to the treatment tank 2 through the reflux pipe 10 during the subsequent stage, whereby the foreign substances can be removed from the treatment liquid 3. To supply the defoamed treatment liquid 3 to either the foreign substance discharge pipe 12 or the reflux pipe 10, a switching valve may be disposed at a branch section between the foreign substance discharge pipe 12 and the reflux pipe 10, or open/close valves may each be disposed in the foreign substance discharge pipe 12 and the reflux pipe 10 downstream of the branch section.

Also, if it is necessary to continuously remove foreign substances, a separator 13 (e.g., filter, cyclone) that separates foreign substances from the defoamed treatment liquid 3 is provided in the reflux pipe 10 as illustrated in FIG. 6. The defoamed treatment liquid 3 from which the foreign substances have been separated and removed is refluxed to the treatment tank 2 through the second reflux pipe 14, and the defoamed treatment liquid 3 containing the foreign substances is discharged outside through the foreign substance discharge pipe 12.

### <Suitable Application Example 1 of Defoaming Device of Present Invention>

The defoaming device of the present invention can be suitably attached to a culture tank (treatment tank) for culturing microorganisms, such as aerobic bacteria and anaerobic bacteria, or cells.

Microorganisms are cultured to produce enzyme agents, fermented foods, biochemicals, biofuels, and other products, and cells are cultured to produce biopharmaceuticals, cultured meat, and other cells. During the process of culturing microorganisms or cells, a large amount of foam is generated at an upper part of the culture solution. Moreover, the foam generated in the process of culturing microorganisms or cells is firm foam (foam with high viscosity, elasticity, and low fluidity) different from the foam of surfactants, such as detergents, and cannot be easily removed because the culture solution contains sugar, proteins, and other components.

A large amount of firm foam generated at an upper part of the culture solution thus causes the following problems a) to c) in the process of culturing microorganisms or cells.
a) The productivity is low because the culture solution can be loaded up to only about 60% of the culture tank to smoothly carry out the culture process in consideration of generation of foam.
b) The defoaming effect is not sufficient even if a foam-cutting blade or other components are installed in the culture tank for the purpose of defoaming.
c) The addition of defoaming agents (e.g., silicone-based, oil-based) for the purpose of defoaming somewhat inhibits the culture of microorganisms or cells.

When the defoaming device of the present invention is attached to a culture tank for culturing microorganisms or cells, the defoaming can be performed efficiently without inhibiting the culture of microorganisms or cells while ensuring cleanliness and sanitation.

Further, in the culture of microorganisms or cells, substances derived from culture materials and by-products (e.g., proteins) produced during the culture reactions can lead to generation of foreign substances in the culture solution. These foreign substances are unnecessary or undesirable for the growth and proliferation of microorganisms or cells, and adhere to the foam. The use of the defoaming device of the present invention can remove these foreign substances from the culture solution.

Also, when the defoaming device of the present invention is used in the culture of aerobic bacteria, an oxygen-containing gas, specifically air, preferably air with an increased oxygen content, can be used as the high-speed gas stream from the viewpoint of avoiding inhibiting the activity of aerobic bacteria. From the viewpoint of avoiding inhibiting the activity of aerobic bacteria, the lower limit of the oxygen concentration is set to 20% or more, preferably 25% or more, more preferably 30% or more. Also, from the viewpoint of preventing oxidative deterioration of the treatment liquid, the upper limit of the oxygen concentration is preferably set to 50% or less.

Also, when the defoaming device of the present invention is used in the culture of anaerobic bacteria, a gas with a low oxygen content, specifically, air with a reduced oxygen content, more preferably a gas comprised mainly of carbon dioxide or nitrogen, more preferably a gas consisting of carbon dioxide or nitrogen, can be used as the high-speed gas stream from the viewpoint of avoiding inhibiting the activity of anaerobic bacteria. From the viewpoint of avoiding inhibiting the activity of anaerobic bacteria, the upper limit of the oxygen concentration is set to 10% or less, preferably 8% or less, more preferably 0.5% or less.

### <Suitable Application Example 2 of Defoaming Device of Present Invention>

The defoaming device of the present invention can be suitably attached to a culture tank (treatment tank) for culturing yeast, particularly a beer fermentation tank (treatment tank). The beer fermentation tank may have various internal volumes. For example, a large fermentation tank as large as 100 m³ is also used.

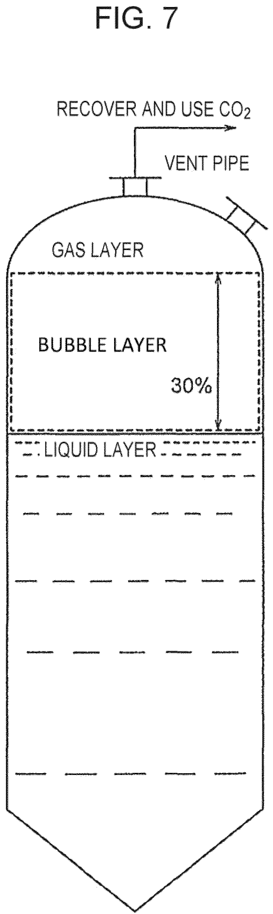

As illustrated in FIG. 7, in the beer fermentation process, wort, yeast, and other ingredients are placed in the fermentation tank, and the wort is decomposed into alcohol and carbon dioxide by the action of the yeast over a period of 7 to 8 days. At a certain point, the foam formed by the generated carbon dioxide reaches 30% of the internal volume of the fermentation tank. Furthermore, the foam formed in the fermentation tank is firm foam (foam with high viscosity, elasticity, and low fluidity) because the culture solution contains carbohydrates and proteins. Therefore, the defoaming device 1, which can be disposed outside the lid part or upper mirror part 5 at the upper part of the treatment tank 2 and is excellent in suction action and defoaming action like the defoaming device of the present invention, can be suitably used.

Further, in beer fermentation, dregs, such as hops and resins, known as "decke" from the fermentation liquid adhere to the foam surface as a result of foaming, and adhere to the inner surface of the fermentation tank in the form of a ring, and remain there. It is thus desirable to remove decke from the fermentation liquid. As illustrated in FIG. 5 and FIG. 6, the decke contained in the defoamed fermentation liquid is discharged outside through the foreign substance discharge pipe 12, whereby decke can be removed from the fermentation liquid during the defoaming process in the fermentation tank.

Furthermore, decke is a bitter ingredient that affects the taste of beer, and the amount of decke allowed to adhere to the inner surface of the fermentation tank or removed has conventionally been determined based on the intuition of skilled brewers. By using the defoaming device 1 illustrated in FIG. 5 and FIG. 6, however, the amount of decke to be removed from the fermentation liquid can be quantitatively adjusted without relying on the intuition of skilled brewers.

Also, when the defoaming device of the present invention is used in the culture of yeast, a carbon dioxide-containing gas can be used as the high-speed gas stream from the viewpoint of avoiding inhibiting the activity of yeast. From an economic standpoint, carbon dioxide released by yeast during fermentation is preferably extracted and reused as carbon dioxide to be contained in the high-speed gas stream. Since the use of a high-speed gas stream with a high oxygen concentration alters the metabolism of yeast and affects the flavor of beer, the concentration of carbon dioxide in the high-speed gas stream is preferably maintained high. Specifically, the lower limit of the carbon dioxide concentration of the high-speed gas stream is set to 80% or more, preferably 90% or more, more preferably 99% or more. Also, the upper limit of the carbon dioxide concentration is preferably set to 100%.

### <Suitable Application Example 3 of Defoaming Device of Present Invention>

The defoaming device of the present invention can be suitably used in the processing of protein-containing dispersion liquids.

In the processing of protein-containing dispersion liquids, such as milk and soy milk, proteins are adsorbed to the interface between air and a protein-containing dispersion liquid, such as milk and soy milk, to form a film, and firm foam (foam with high viscosity, elasticity, and low fluidity) is formed on the surface by agitation and ventilation. When the defoaming device of the present invention is used in the processing of protein-containing dispersion liquids, such as milk and soy milk, a gas with a low oxygen content, specifically air, preferably air with a reduced oxygen content, more preferably a gas consisting of nitrogen, can be used as the high-speed gas stream from the viewpoint of preventing oxidative deterioration of protein-containing dispersion liquids, such as milk and soy milk. From the viewpoint of preventing oxidative deterioration of protein-containing dispersion liquids, such as milk and soy milk, the upper limit of the oxygen concentration is set to 21% or less, preferably 15% or less, more preferably 1% or less.

### <First Embodiment of Treatment Apparatus of Present Invention>

A treatment apparatus of the present invention includes the defoaming device of the present invention and exhibits the same excellent effects as the defoaming device of the present invention.

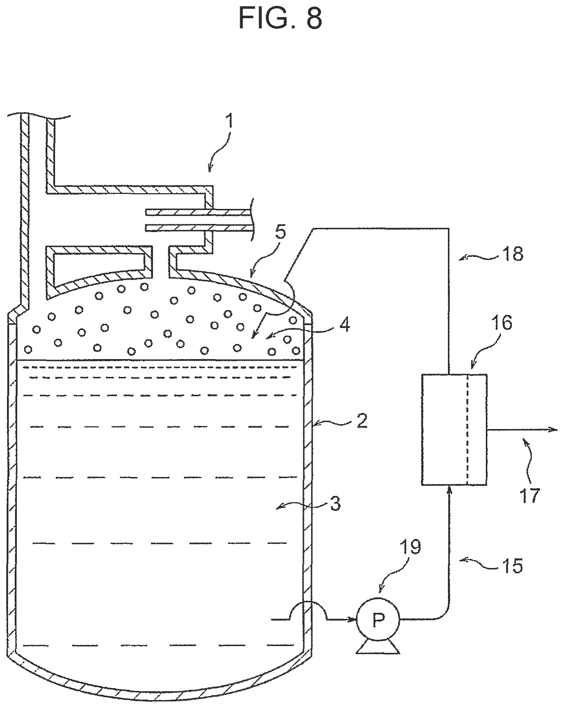

In a first embodiment of the treatment apparatus of the present invention, as illustrated in FIG. 8, a treatment apparatus includes a treatment tank 2 that accommodates and treats a treatment liquid 3. In this treatment apparatus, the defoaming device 1 of the present invention is disposed outside a lid part or upper mirror part 5 of the treatment tank 2 and suctions foam 4 generated at the upper part of the treatment liquid 3 in the treatment tank 2 to remove the foam 4.

When this treatment apparatus is used particularly for culturing aerobic bacteria, the treatment tank 2 preferably includes an agitator (not shown) for agitating the treatment liquid 3 to supply enough oxygen to aerobic bacteria.

In the first embodiment, the treatment apparatus can include a) an extraction conduit 15 through which the treatment liquid 3 extracted from the treatment tank 2 is supplied to a filter 16 by using a culture tank pump 19, b) a recovery conduit 17 through which a filtrate separated by the filter 16 is recovered, and c) a reflux conduit 18 through which the concentrate separated by the filter 16 and free of the filtrate is refluxed to the treatment tank 2. The filtration membrane of the filter 16 may be an organic membrane or an inorganic membrane, and the filtration membrane may have any shape, such as a flat membrane, a hollow fiber membrane, or a spiral type. In particular, a hollow fiber membrane module is preferred.

### <Second Embodiment of Treatment Apparatus of Present Invention>

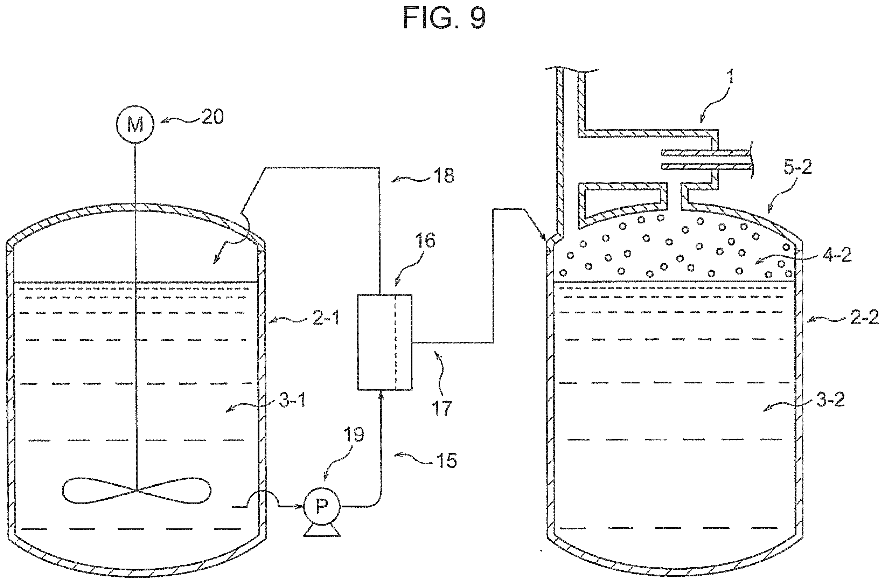

In a second embodiment of the treatment apparatus of the present invention, as illustrated in FIG. 9, the treatment apparatus includes a primary treatment tank 2-1 that accommodates and treats a treatment liquid 3-1 and a secondary treatment tank 2-2 that treats a filtrate 3-2 obtained by extracting the treatment liquid 3-1 from the primary treatment tank 2-1 and filtering the extracted treatment liquid 3-1. In this treatment apparatus, the defoaming device 1 of the present invention is disposed outside a lid part or upper mirror part 5-2 of the secondary treatment tank 2-2 and suctions foam 4-2 generated at an upper part of the filtrate 3-2 in the secondary treatment tank 2-2 to remove the foam 4-2. As also described in the first embodiment, the filtrate 3-2 in the secondary treatment tank 2-2 is obtained by extracting the treatment liquid 3 from the primary treatment tank 2-1 into a filter 16 through an extraction conduit 15 using a culture tank pump 19, and supplying the filtrate 3-2 separated by the filter 16 to the secondary treatment tank 2-2 through a recovery conduit 17. It should be noted that the concentrate separated by the filter 16 and free of the filtrate 3-2 is refluxed to the primary treatment tank 2-1 through a reflux conduit 18.

When this treatment apparatus is used particularly for culturing aerobic bacteria, the primary treatment tank 2-1 preferably includes an agitator 20 for agitating the treatment liquid 3-1 to supply enough oxygen to aerobic bacteria.

### <Defoaming Method of Present Invention>

A defoaming method of the present invention uses the defoaming device of the present invention and exhibits the same excellent effects as the defoaming device of the present invention.

### Examples 1 to 19, Comparative Examples 1 to 5

The defoaming device illustrated in FIG. 1 and FIG. 2 was used to evaluate the suction effect, defoaming effect, and entrainment on the basis of experiments.

### The treatment liquids used in the experiments are as described below.

<Treatment Liquid 1> Soy milk (water containing 0.4 mass% proteins, 0.1 mass% carbohydrates, 0.3% lipids, and other components)
<Treatment Liquid 2> Bacteria culture solution (medium containing bacteria, 5 mass% carbohydrates, a nitrogen source such as soy protein, ammonium sulfate, minerals, and other components)
   The evaluation criteria for the suction effect, defoaming effect, and entrainment are described below.
<Suction Effect>
   ○: The foam 4 is sufficiently suctioned into the defoaming pipe 6 through the suction pipe 8.
   △: The foam 4 is not sufficiently suctioned into the defoaming pipe 6.
   x: The foam 4 is not suctioned into the defoaming pipe 6.
<Defoaming Effect>
   ○: No foam 4 remains at an end of the defoaming pipe 6 on the downstream side of the high-speed gas stream A.
   △: Part of the foam 4 remains at the end of the defoaming pipe 6 on the downstream side of the high-speed gas stream A.
   x: All of the foam 4 remains at the end of the defoaming pipe 6 on the downstream side of the high-speed gas stream A.
<Entrainment>
   ○: There are no droplets of the defoamed treatment liquid above the branch section of the gas discharge pipe 9.
   △: There are some droplets of the defoamed treatment liquid above the branch section of the gas discharge pipe 9.
   x: There significantly are droplets of the defoamed treatment liquid above the branch section of the gas discharge pipe 9.

It should be noted that the flow rate (m/s) is the flow rate of the high-speed gas stream A along the center line of the defoaming pipe 6 at a midpoint between the end of the gas supply pipe 7 on the downstream side of the high-speed gas stream A and the end of the defoaming pipe 6 on the downstream side of the high-speed gas stream A.

The results of Examples 1 to 19 and Comparative Examples 1 to 5 are summarized in Table 1 below.

**[Table 1]**

| | Treatment liquid | a (mm) | b (mm) | a/b | Flow rate (m/s) | Suction effect | Defoaming effect | Entrainment |
|---|---|---|---|---|---|---|---|---|
| Example 1 | | 17 | 10 | 1.7 | 10 | Δ | Δ | ○ |
| Example 2 | | 17 | 10 | 1.7 | 15 | Δ | ○ | ○ |
| Example 3 | | 17 | 10 | 1.7 | 30 | ○ | ○ | ○ |
| Example 4 | | 17 | 10 | 1.7 | 70 | ○ | ○ | ○ |
| Example 5 | | 17 | 10 | 1.7 | 90 | ○ | ○ | Δ |
| Example 6 | | 12 | 6 | 2.0 | 25 | ○ | ○ | ○ |
| Example 7 | | 12 | 8 | 1.5 | 60 | ○ | ○ | ○ |
| Example 8 | | 22.2 | 7.5 | 3.0 | 25 | ○ | ○ | ○ |
| Example 9 | | 22.2 | 7.5 | 3.0 | 30 | ○ | ○ | Δ |
| Example 10 | | 22.2 | 5 | 4.4 | 25 | ○ | ○ | ○ |
| Example 11 | treatment liquid 1 (soy milk) | 22.2 | 5 | 4.4 | 30 | ○ | ○ | Δ |
| Comparative Example 1 | | 17 | 10 | 1.7 | 5 | Δ | x | ○ |
| Comparative Example 2 | | 12 | 12 | 1.0 | 15 | x | not observed | not observed |
| Comparative Example 3 | | 22.2 | 4 | 5.6 | 25 | x | ○ | Δ |
| Comparative Example 4 | | 22.2 | 4 | 5.6 | 30 | x | ○ | Δ |
| Example 12 | | 17 | 10 | 1.7 | 15 | ○ | ○ | ○ |
| Example 13 | | 17 | 10 | 1.7 | 30 | ○ | ○ | ○ |
| Example 14 | | 17 | 10 | 1.7 | 70 | ○ | ○ | ○ |
| Example 15 | treatment liquid 2 (bacteria culture solution) | 17 | 10 | 1.7 | 90 | ○ | ○ | Δ |
| Example 16 | | 11 | 6 | 1.8 | 70 | ○ | ○ | ○ |
| Example 17 | | 11 | 6 | 1.8 | 80 | ○ | ○ | ○ |
| Example 18 | | 11 | 6 | 1.8 | 110 | ○ | ○ | Δ |
| Example 19 | | 11 | 6 | 1.8 | 130 | ○ | ○ | Δ |
| Comparative Example 5 | | 11 | 6 | 1.8 | 140 | ○ | ○ | x |

It should be noted that in Table 1 above, "not observed" means that a <defoaming effect> and a <entrainment> were not observed because the foam 4 was not suctioned into the defoaming pipe 6 through the suction pipe 8.

Table 1 shows that, when the lower limit of the flow rate of the high-speed gas stream A in the defoaming pipe 6 is set to 10 m/s or more, preferably 15 m/s or more, more preferably 30 m/s or more in the first embodiment of the defoaming device of the present invention, it is possible to immediately break down even firm foam (foam with high viscosity, elasticity, and low fluidity) containing proteins, carbohydrates, lipids, and other components.

Also, when the upper limit of the flow rate of the high-speed gas stream A in the defoaming pipe 6 is set to 130 m/s or less, preferably 90 m/s or less, more preferably 70 m/s or less, it is possible to prevent the defoamed treatment liquid from being entrained as droplets in the high-speed gas stream A.

Also, when the lower limit of the ratio (a/b) of the inner diameter a of the defoaming pipe 6 to the inner diameter b of the gas supply pipe 7 is 1.1 or more, preferably 1.5 or more, more preferably 1.7 or more, still more preferably 1.8 or more, yet still more preferably 2.0 or more, it is possible to sufficiently suction even firm foam (foam with high viscosity, elasticity, and low fluidity) containing proteins, carbohydrates, lipids, and other components.

Also, when the upper limit of the ratio (a/b) of the inner diameter a of the defoaming pipe 6 to the inner diameter b of the gas supply pipe 7 is 4.5 or less, preferably 4.0 or less, more preferably 3.5 or less, still more preferably 3.0 or less, it is possible to sufficiently suction even firm foam (foam with high viscosity, elasticity, and low fluidity) containing proteins, carbohydrates, lipids, and other components.

Examples 1 to 19 and Comparative Examples 1 to 5 in Table 1 demonstrate the operational advantages of the first embodiment of the defoaming device of the present invention. The second embodiment of the defoaming device of the present invention slightly differs in structure from the first embodiment of the defoaming device but obviously exhibits the same operational advantages as the first embodiment of the defoaming device of the present invention because the second embodiment of the defoaming device is based on the same principle and action of suctioning the foam 4 from the treatment tank 2 through the suction pipe 8 by means of the ejector action of the high-speed gas stream A passing inside the defoaming pipe 6 and breaking down the suctioned foam 4 by means of the shearing action of the high-speed gas stream A.

### Description of the Symbols

1 Defoaming device
2 Treatment tank
2-1 Primary treatment tank
2-2 Secondary treatment tank
3, 3-1 Treatment liquid
3-2 Filtrate
4 Foam (of treatment liquid)
4-2 Foam (of filtrate)
5 Lid part or upper mirror part (at upper part of treatment tank)
5-2 Lid part or upper mirror part (of secondary treatment tank)
6 Defoaming pipe
7 Gas supply pipe
8 Suction pipe
8-1 Connection opening of suction pipe to defoaming pipe
9 Gas discharge pipe
10 Reflux pipe
11 Connection part (of defoaming pipe to suction pipe)
12 Foreign substance discharge pipe
13 Separator
14 Second reflux pipe
15 Extraction conduit
16 Filter
17 Recovery conduit
18 Reflux conduit
19 Culture tank pump
20 Agitator
A High-speed gas stream
a Inner diameter of defoaming pipe
b Inner diameter of gas supply pipe
c Inner diameter of connection part (of defoaming pipe to suction pipe)
30 Culture tank
31 Aerobic bacteria culture solution
32 Foam
33 Exhaust pipe
34 Foam sensor
35 Ultrasonic horn
36 Reflux liquid pipe
37 Agitator
38 Cyclone
41 Culture tank
41a Lid part or upper mirror part
42 Microorganism culture solution
44 Foam
45 Ultrasonic irradiation unit
51 Steel strip
55 Washing tank
59 Steam ejector
510 Defoaming box
511 Defoaming plate
518 Foam
61 Gas supply pipe
63 Suction pipe
64 Defoaming pipe
612 Steel strip
613 Washing tank
617 Foam
6101 Defoaming device

## Claims

1. A defoaming device that suctions and removes foam generated at an upper part of a treatment liquid in a treatment tank, **characterized in that**
the defoaming device is disposed outside a lid part or upper mirror part at an upper part of the treatment tank,
the defoaming device includes a) a defoaming pipe inside which a high-speed gas stream passes, b) a gas supply pipe through which the high-speed gas stream is supplied to the defoaming pipe, and c) a suction pipe that is connected at one end to the defoaming pipe and at the other end to the lid part or upper mirror part and through which the foam is suctioned from the treatment tank,
a gas discharge pipe through which gas is discharged outside and a reflux pipe through which the defoamed treatment liquid is refluxed to the treatment tank are connected to an end of the defoaming pipe on a downstream side of the high-speed gas stream,
the high-speed gas stream in the defoaming pipe has a flow rate of 10 m/s or more and 130 m/s or less, and a ratio (a/b) of an inner diameter a of the defoaming pipe to an inner diameter b of the gas supply pipe is 1.1 or more and 4.5 or less, and
the foam is suctioned from the treatment tank through the suction pipe by means of an ejector action of the high-speed gas stream passing inside the defoaming pipe, and the suctioned foam is broken down by means of a shearing action of the high-speed gas stream.

2. The defoaming device according to Claim 1, **characterized in that** an end of the gas supply pipe on the downstream side of the high-speed gas stream is disposed so as to protrude on a further downstream side of the high-speed gas stream than is a connection opening of the suction pipe to the defoaming pipe.

3. A defoaming device that suctions and removes foam generated at an upper part of a treatment liquid in a treatment tank, **characterized in that**
the defoaming device is disposed outside a lid part or upper mirror part at an upper part of the treatment tank,
the defoaming device includes a) a defoaming pipe inside which a high-speed gas stream passes, b) a gas supply pipe through which the high-speed gas stream is supplied to the defoaming pipe, and c) a suction pipe that is connected at one end to the defoaming pipe and at the other end to the lid part or upper mirror part and through which the foam is suctioned from the treatment tank,
a gas discharge pipe through which gas is discharged outside and a reflux pipe through which the defoamed treatment liquid is refluxed to the treatment tank are connected to an end of the defoaming pipe on a downstream side of the high-speed gas stream,
a central portion of a connection part of the defoaming pipe to the suction pipe has a cylindrical shape in a flow direction of the high-speed gas stream, and both outer side portions of the connection part have conical shapes that gradually expand in diameter toward upstream and downstream sides of the high-speed gas stream,
the high-speed gas stream in the defoaming pipe has a flow rate of 10 m/s or more and 130 m/s or less, and a ratio (a/c) of an inner diameter a of the defoaming pipe to an inner diameter c of the connection part is 1.1 or more, and
the foam is suctioned from the treatment tank through the suction pipe by means of an ejector action of the high-speed gas stream passing inside the defoaming pipe, and the suctioned foam is broken down by means of a shearing action of the high-speed gas stream.

4. The defoaming device according to any one of Claims 1 to 3, **characterized in that** an extension pipe that extends the suction pipe toward a liquid surface of the treatment liquid is connected to the suction pipe.

5. The defoaming device according to any one of Claims 1 to 3, **characterized in that** the gas discharge pipe has a mist separator that prevents droplets from being released outside.

6. The defoaming device according to any one of Claims 1 to 3, **characterized in that** a foreign substance discharge pipe through which foreign substances are discharged outside is connected to the reflux pipe.

7. The defoaming device according to any one of Claims 1 to 3, **characterized in that** a separator that separates foreign substances from the defoamed treatment liquid is disposed in the reflux pipe, and a second reflux pipe through which the defoamed treatment liquid from which the foreign substances have been separated and removed is refluxed to the treatment tank and a foreign substance discharge pipe through which the foreign substances are discharged outside are connected to the separator.

8. The defoaming device according to any one of Claims 1 to 3, **characterized in that** the treatment liquid is a culture solution in which microorganisms or cells are cultured.

9. The defoaming device according to any one of Claims 1 to 3, **characterized in that** the treatment liquid is a culture solution in which aerobic bacteria are cultured, and the high-speed gas stream contains oxygen.

10. The defoaming device according to any one of Claims 1 to 3, **characterized in that** the treatment liquid is a culture solution in which anaerobic bacteria are cultured, and the high-speed gas stream has a low oxygen content.

11. The defoaming device according to any one of Claims 1 to 3, **characterized in that** the treatment liquid is a culture solution in which yeast is cultured to produce beer, and the high-speed gas stream contains carbon dioxide.

12. The defoaming device according to any one of Claims 1 to 3, **characterized in that** the treatment liquid is a protein-containing dispersion liquid, and the high-speed gas stream has a low oxygen content.

13. A treatment apparatus comprising a treatment tank that accommodates and treats a treatment liquid, **characterized in that** the defoaming device according to any one of Claims 1 to 3 is disposed outside a lid part or upper mirror part of the treatment tank.

14. The treatment apparatus according to Claim 13, **characterized in that** the treatment tank has an agitator for agitating the treatment liquid.

15. The treatment apparatus according to Claim 13, **characterized in that** the treatment apparatus includes a) an extraction conduit through which the treatment liquid is extracted from the treatment tank and supplied to a filter, b) a recovery conduit through which a filtrate separated by the filter is recovered, and c) a reflux conduit through which a concentrate separated by the filter and free of the filtrate is refluxed to the treatment tank.

16. A treatment apparatus comprising a primary treatment tank that accommodates and treats a treatment liquid and a secondary treatment tank that treats a filtrate obtained by extracting the treatment liquid from the primary treatment tank and filtering the extracted treatment liquid, **characterized in that** the defoaming device according to any one of Claims 1 to 3 is disposed outside a lid part or upper mirror part of the secondary treatment tank.

17. The treatment apparatus according to Claim 16, **characterized in that** the primary treatment tank has an agitator for agitating the treatment liquid.

18. A defoaming method for removing foam generated at an upper part of a treatment liquid in a treatment tank, **characterized by** comprising using the defoaming device according to any one of Claims 1 to 3 disposed outside a lid part or upper mirror part of the treatment tank to remove the foam generated at the upper part of the treatment liquid.
